# EUROPÄISCHE PATENTSCHRIFT

(11) **EP 3 585 324 B1**
(45) Veröffentlichungstag und Bekanntmachung des Hinweises auf die Patenterteilung: **12.08.2020**
(21) Anmeldenummer: 18733937.9
(22) Anmeldetag: 14.05.2018
(51) Int. Cl.: A61F 5/11

(54) **KIT ZUR NAGELKORREKTUR**
NAIL CORRECTION KIT
KIT DE CORRECTION D'ONGLE

(30) Priorität: 12.05.2017 DE 102017004546
(43) Veröffentlichungstag der Anmeldung: 01.01.2020
(73) Patentinhaber: L/N Health and Beauty ApS, 8220 Lystrup (DK)
(72) Erfinder: HØJBERG, Diana, 8240 Risskov (DK)
(74) Vertreter: Jungblut & Seuss
(86) Internationale Anmeldenummer: PCT/IB2018/053356
(87) Internationale Veröffentlichungsnummer: WO 2018/207164

(56) Entgegenhaltungen:
- EP-A1- 2 829 255
- WO-A1-2011/039243
- DE-A1- 4 446 650
- Gary Alex: "Universal Adhesives: The Next Evolution in Adhesive Dentistry?", Compendium of continuing education in densitry, Vol. 36, Number 1, 15. Januar 2015 (2015-01-15), Seiten 15-26, XP055503970, Gefunden im Internet: URL:http://drgaryalex.com/wp-content/uploa ds/2015/08/1-Uniiversal-Adhesives-THe-next -Evolution-in-Adhesive-Dentistry.pdf [gefunden am 2018-09-03]

## Beschreibung

Die vorliegende Erfindung betrifft ein Kit zur Durchführung eines Nagelkorrekturverfahrens für einen menschlichen oder tierischen Fuß- oder Fingernagel. Die Anmeldung nimmt die Priorität der Voranmeldung DE 102017004546.1 (Anmeldetag: 12.5.2017) in Anspruch.

Es sind verschiedene Methoden bekannt, um Fuß- oder Fingernägel zu korrigieren. Derartige Nägel weisen häufig eine starke Krümmung auf, die dazu führen, dass sie ins Nagelbett drücken oder einwachsen. Eine häufige Behandlungsmethode besteht darin, den gesamten Nagel chirurgisch zu entfernen. Die Behandlung ist nicht nur schmerzhaft, sondern führt auch häufig nicht zur Nagelkorrektur: Der neu nachwachsende Nagel ist häufig ebenso gekrümmt, wie der ursprüngliche Nagel.

Eine Alternative, nicht-chirurgische Behandlungsmethode besteht darin, eine Nagelkorrekturspange an den betroffenen Nagel anzubringen. Eine derartige Spange ist im Rahmen der deutschen Patentschrift DE 10 2005 039 147 B3 beschrieben. Die Spange greift dabei unter den Nagel, was häufig zu Gewebereizungen führt. Im Rahmen der WO 2011/039243 A1 wird ein Mehrschichtenpolymersystem beschrieben, welches durch gegenläufige Kontraktion und Expansion verschiedener Schichten zu seitlichen Zugkräften auf den Nagel führen soll, mit der Folge einer mechanischen Korrektur. Wie sich herausgestellt hat, ist das in der WO 2011/039243 A1 beschriebene Verfahren nicht nacharbeitbar: Alle Versuche, ein derartiges mehrschichtiges System nachzuarbeiten, welches einerseits auf dem Nagel hält und andererseits die nötigen Korrekturkräfte entwickelt blieben erfolglos.

Es besteht daher weiterhin Bedarf an Hilfsmitteln für die Korrektur fehlgewachsener Fuß- oder Fingernägel.

Überraschenderweise wurde gefunden, dass das nachfolgende beschriebene Kit zur Nagelkorrektur die vorstehenden Nachteile überwindet und eine Korrektur eingewachsener Finger- oder Fußnägel auf einfache Weise ermöglicht. Je nach Grad der Verformung der Nägel (insbesondere des Krümmungsgrades) können unterschiedliche Zusammensetzungen auf Basis von photopolymerisierbaren Komponenten zur Anwendung kommen. Optional können zusätzlich ein oder mehrere metallische Federspangen verwendet werden. Durch die Art der Anbringung der metallischen Federspangen ist ausgeschlossen, dass es zu mechanischen Gewebereizungen kommt.

Das erfindungsgemäße Kit zur Nagelkorrektur enthält
a) Grundierungsmittel (Primer), enthaltend

| | |
|---|---|
| 40-60% | Hydroxyethylenmethacrylat |
| 40-60% | Phosphatdimethacrylat, |
| 0,1-1,0 % | Starter, |

b) mindestens eine Zusammensetzung zur Herstellung einer lichthärtenden Nagelspange, enthaltend

| | |
|---|---|
| 15 -45 % | Bisphenol-(A)-(di)methacrylat, Urethan-dimethacrylat im Verhältnis 1:5 bis 5:1, |
| 85 - 55% | Füllstoffe und Pigmente |
| 0,1-1 % | Campherchinon, Aminostarter, |

und
c) optional ein oder mehrere Federspangen.

Die Komponente a des erfindungsgemäßen Kits ist ein Grundiermittel (Primer), das als Haftvermittler wirkt. Im Rahmen der Untersuchungen zur vorliegenden Erfindung hat sich herausgestellt, dass die Haftung des Photopolymers auf dem Nagel kritisch für den Erfolg des Korrekturverfahrens ist. Es zeigt sich, dass die Haftung des Photopolymers auf dem Nagel signifikant verbessert wird, wenn der im Rahmen der Erfindung beschriebene Haftvermittler als Grundiermittel eingesetzt wird. Dieser Haftvermittler enthält 40 - 60 % Hydroxyethylenmethacrylat und 40 - 60 % Phosphatdimethacrylat sowie 0,1 - 1,0 % Polymerisationsstarter. Die Polymerisationsstarter werden unten näher beschrieben. Das Mischungsverhältnis kann im Rahmen der oben genannten Prozentangaben variieren. Vorteilhafterweise sind die beiden Methacrylate in ungefähr gleichem Verhältnis enthalten. Es versteht sich von selbst, dass alle Komponenten zusammen 100 % ergeben.

Die eigentliche Nagelspange wird mittels des photopolymerisierbaren Materials b gebildet. Es handelt sich dabei um eine Zusammensetzung enthaltend

| | |
|---|---|
| 15 -45 % | Bisphenol-(A)-(di)methacrylat, Urethan-dimethacrylat im Verhältnis 1:5 bis 5:1, |
| 85 - 55% | Füllstoffe und Pigmente |
| 0,1-1 % | Campherchinon, Aminostarter. |

Der oben genannte Zusammensetzungsbereich erlaubt eine unterschiedliche Einstellung der mechanischen Eigenschaften, in Form unterschiedlicher Härtegrade. In der Praxis hat es sich bewährt, zwei Zusammensetzungen zur Verfügung zu stellen, von denen eine vergleichsweise weich, die andere vergleichsweise hart ist.

Die weiche Zusammensetzung enthält beispielsweise

| | |
|---|---|
| 17 -21 % | Bisphenol-(A)-(di)methacrylat, Urethan-dimethacrylat im Verhältnis 1:4 bis 4:1, |
| 79 - 83 % | Füllstoffe und Pigmente |
| 0,1-1 % | Campherchinon, Aminostarter. |

Die harte Zusammensetzung enthält beispielsweise

| | |
|---|---|
| 36 -40 % | Bisphenol-(A)-(di)methacrylat, Urethan-dimethacrylat im Verhältnis 1:4 bis 4:1, |
| 60 - 64 % | Füllstoffe und Pigmente |
| 0,1-1 % | Campherchinon, Aminostarter. |

Die in den Zusammensetzungen enthaltenden Monomere bis Bisphenol-(A)-(di)-methacrylat und Urethan-dimethacrylat sind bevorzugt in etwa gleicher Menge enthalten. Mischungsverhältnisse, bei denen die beiden Hauptkomponenten im Massenverhältnis zwischen 1:5 und 5:1 enthalten sind generell geeignet. Besonders vorzugswürdig sind Mischungsverhältnisse, bei denen die beiden Hauptkomponenten im Massenverhältnis zwischen 1:2 und 2:1 enthalten sind.

Das Material für die lichthärtende Nagelspange enthält ferner Füllstoffe und Pigmente. Hierbei handelt es sich um Füllstoffe auf Silikatbasis (Silicafiller), auf der Basis von gemahlenem Bariumglas (Bariumglasfiller) sowie Polymerpartikel. Die partikulären Zusätze haben Durchmesser im Bereich von 0,1-10 µm, vorzugsweise sind sie kleiner als 5 µm. Insbesondere die Füllstoffe auf der Basis von Bariumglas tragen erheblich zu den mechanischen Eigenschaften der Nagelspange bei. Zusätzlich können noch Pigmente in der Zusammensetzung enthalt sein, um der gebildeten Nagelspange ein ästhetisches Äußeres zu geben. Auch die Pigmente sollten vorzugsweise Durchmesser im Bereich von 0,1-10 µm aufweisen.

Die erfindungsgemäßen Zusammensetzungen enthalten ferner Polymerisationsstarter, die durch Bestrahlung mit Licht die gewünschte Polymerisationsreaktion auslösen können. Hierzu sind im Prinzip alle klassischen Polymerisationsstarter geeignet. Als besonders geeignet hat sich die Kombination Campherchinon mit Aminostartern, nämlich tertiären Aminen (z. B. Triethanolamin, N,N-Dimethyl-p-toluidin, Triethylamin, 4-Dimethylaminobezoesäureethylester, N,N-Tetramethylanilin) herausgestellt. Alternativ kann beispielsweise 2-Ethyl-Anthrachinon in Kombination mit N-Phenyl Glycin verwendet werden. Bei Verwendung eines derartigen Systems kann die fertige Zusammensetzung überraschenderweise über längere Zeit gelagert werden, ohne dass eine Polymerisationsreaktion eintritt. Erst nach Beleuchtung mit einer angepassten Lichtquelle tritt die Polymerisationsreaktion ein. Hierzu kann z.B. eine LED-Lichtquelle mit einer Wellenlänge von ca. 450 nm und einer Lichtleistung von 1000-1500mW/cm² verwendet werden.

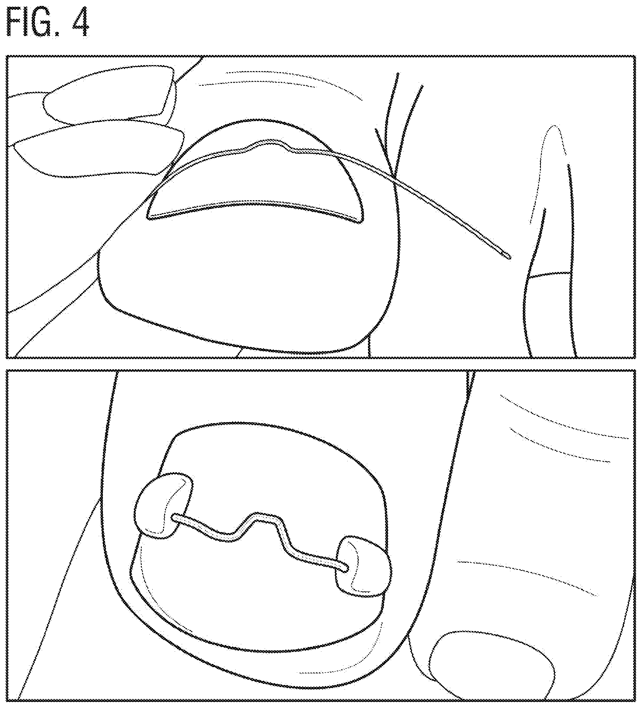

Optional enthält das erfindungsgemäße Kit darüber hinaus noch eine oder mehrere Spangen aus Federstahl. Diese sind aus einem Federstahldraht gefertigt mit einem Durchmesser von 0,3 - 1,5 mm. Die Spange kann ein oder mehrere U-förmige Schlafen aufweisen. Die Metallfederspange wird mit dem oben beschriebenen lichthärtenden Material oben auf den betroffenen Nagel aufgebracht (aufgeklebt). Im Unterschied zu vorbeschriebenen Therapieverfahren unter Verwendung derartiger Metallspangen ist es weder erforderlich den Nagel an der Außenseite zu umschließen, noch sind Bohrungen in dem Nagel notwendig. Figur 4 zeigt eine erfindungsgemäße Befestigung einer derartigen Metallspange.

### Das erfindungsgemäße Kit wird wie folgt verwendet:

Der Therapeut hebt zunächst den betroffenen Nagel vom Nagelbett ab. Im Rahmen dieser Prozedur kann ein Stoffstreifen zwischen Nagel und Nagelbett eingeschoben werden. Der Nagel wird dann zunächst mit dem Grundierungsmittel (Primer) des Kits behandelt. Dabei ist insbesondere darauf zu achten, dass der Nagel trocken ist. Der betroffene Nagel sollte insbesondere in den 24 Stunden vor der Auftragung nicht länger mit Wasser in Kontakt gewesen sein. Der Patient sollte beispielsweise nicht gebadet haben. Ein kurzes Waschen oder Duschen ist hingegen unschädlich, sofern der Nagel wieder gut getrocknet wurde. Erforderlichenfalls kann der Nagel mit einem Heißluftgebläse getrocknet werden. Der Primer wird zunächst auf die notwendigen Stellen aufgetragen. Im Regelfall empfiehlt es sich, den Primer flächig auf den gesamten Nagel aufzutragen. Nach dem Auftragen wird mittels einer Lichtquelle (bevorzugt blaues Licht mit ca. 450 nm und 100mW/cm²) die Polymerisation gestartet. Bei Verwendung einer üblichen Lichtquelle ist die Polymerisation nach einem Zeitraum von 5 Sekunden bis 60 Sekunden abgeschlossen, im Regelfall ist eine 10-sekündige Belichtung ausreichend.

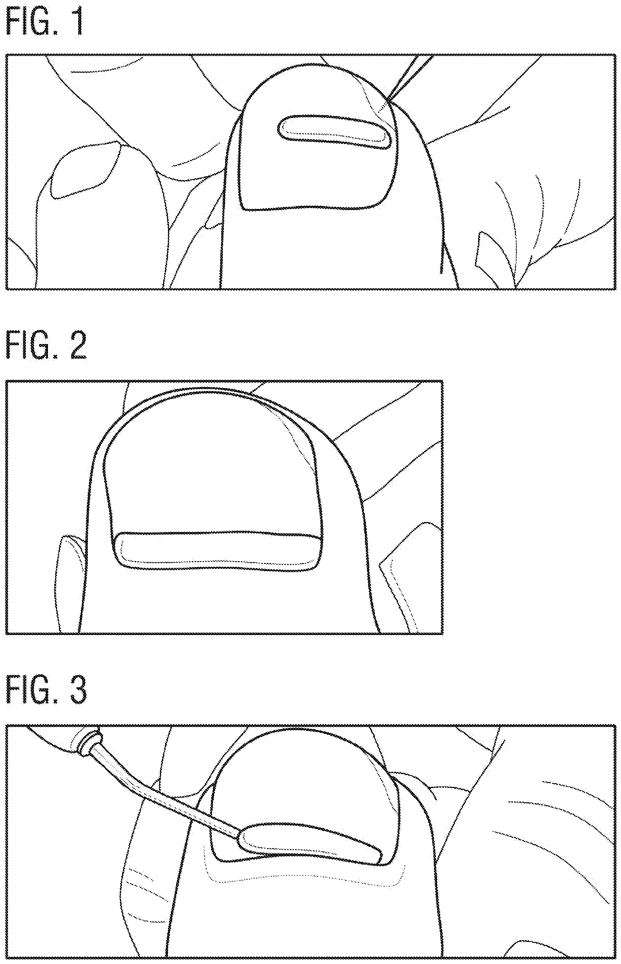

Anschließend wird die lichthärtende Nagelspange aufgetragen. Dabei wird der Nagel vom Therapeuten mittels eines Werkzeuges in der gewünschten Stellung gehalten. Die Auftragung erfolgt strichförmig (siehe Figur 1) mit einer Strichbreite von 2 - 6 mm. Nach der Auftragung wird bevorzugt unmittelbar die Polymerisation durch Beleuchten mit der oben genannten Lichtquelle gestartet. Es ist wichtig, hierbei den Nagel in der gewünschten Form zu halten. Auch dieser Schritt ist üblicherweise nach einem Zeitraum von 5 - 60 Sekunden abgeschlossen. Danach kann das Material nochmals nachgeschliffen werden, damit keine Kanten entstehen, an denen Gewebe (z. B. Strümpfe) hängen bleiben könnten. Bei stark verformten bzw. sehr stark verdickten Nägeln können mehrere derartige strichförmige Auftragungen vorgenommen werden. Gegebenenfalls können sehr dicke Nägel auch vorbereitend abgeschliffen werden, damit der Nagel wieder verformbar wird. Das Abschleifen muss natürlich vor der Grundierung mit dem Primer erfolgen. Das Photopolymer kann auch entsprechend dicker aufgetragen werden. Im Extremfall kann eine metallische Federspange aufgetragen werden. Hierzu wird zunächst ein Tropfen des lichthärtenden Materials punktförmig aufgetragen und die Federspange in diesen Tropfen eingepresst (Figur 4). Nach Lichthärtung des Tropfens wird die Federspange über den Nagel gespannt und mittels eines zweiten Tropfens des photopolymerisierenden Materials befestigt. Die beiden Enden der Metallspange sollten dabei jeweils aus den Tropfen heraus sichtbar sein. Auch bei dieser Ausführungsform ist eine Vorbereitung des Nagels durch den Primer erforderlich, da ansonsten die Haltbarkeit des Konstruktes auf dem Nagel nicht gewährleistet ist.

Mit dem erfindungsgemäßen Kit ist es auch möglich eine weitere Korrektur eines bereits mit einer Metallspange behandelten Nagels zu ermöglich. Insbesondere im Fall einer Gewebereizung (Nagelbettreizung) durch die klassische Metallspange, kann die klassische Metallspange entfernt und der Nagel mit dem erfindungsgemäßen Kit weiterbehandelt werden.

Die im Rahmen des Kits bereitgestellten Zusammensetzungen werden vorzugsweise in entsprechend angepassten Behältern angeboten. Für den Primer eignen sich grundsätzlich Fläschchen aus Glas oder Kunststoff mit einem Applikationspinsel oder einer Applikationsbürste. Die Zusammensetzungen zur Herstellung von lichthärtenden Nagelspangen sind typischerweise stärker viskos und werden vorzugsweise in Kartuschen zur Nutzung zusammen mit einer Kartuschenpresse oder -pistole angeboten. Alle Behältnisse sind vorzugsweise lichtundurchlässig.

Mit dem erfindungsgemäßen Kit wird das notwendige Material zur Verfügung gestellt, um eingewachsene Fuß- bzw. Fingernägel bei Mensch oder Tier zu korrigieren, ohne dass die im Stand der Technik bekannten Nachteile auftreten. In den meisten Fällen kann die Nagelkorrektur ohne Zuhilfenahme metallischer Spangen erfolgen. Die erfindungsgemäße Zusammensetzung gewährleistet insbesondere eine deutliche bessere Haftung am Nagel, als bekannte Zusammensetzungen. Darüber hinaus können die Vorteile eines Polymers mit denen einer Metallspange kombiniert werden, ohne dass die Nachteile auftreten, die bei früheren Metallspangen häufig auftraten, insbesondere mechanische Reizungen des Nagelbettes.

In einer erfindungsgemäßen Weiterentwicklung der Zusammensetzung enthält der Primer zusätzlich noch ein oder mehrere Antimykotika. Wie sich in der Praxis zeigt, sind korrekturbedürftige Nägel häufig noch mit Nagelpilzen befallen, die zusätzlich Probleme bereiten. Nagelpilze sind bekanntlich schwer behandelbar. Es hat sich herausgestellt, dass die Nagelpilzbehandlung gelingt, wenn dem Primer noch zusätzlich ein oder mehrere Antimykotika zugesetzt werden. Die Beimischung beträgt in der Regel 0,1 - 2 %, vorzugsweise 0,5-1%. Als Antimykotikum sind generell Verbindungen geeignet, die zur Behandlung von Onychomykosen zugelassen sind, wie beispielsweise Econazole, Bifonazole, Chlodrimazole, Fenticonazole, Ketocanazole, Miconazole, Oxiconazole und verwandte Verbindungen.

Bei der Ausführungsform der Erfindung mit Antimykotikazusatz hat sich der ergänzende Zusatz von Penetrationsverstärkern bewährt. Hierzu können die üblichen Penetrationsverstärker zur Nagelpenetration verwendet werden. Besonders bewährt haben sich im Rahmen der vorliegenden Erfindung Penetrationsverstärken auf der Basis substituierter 1,3-Dioxolanen, 1,5-Dioxanen und -acetalen, insbesondere die unter der Marke SEPA® vertriebenen Substanzen und Substanzmischungen.

### Beispiele

Die Erfindung wird durch die nachfolgend beispielhaft gezeigten Zusammensetzungen weiter erläutert:

### A) Grundierungsmittel (Primer)

| | **A1** | **A2** | **A3** | **A4** |
|---|---|---|---|---|
| **Komponente** | (Gew. %) | (Gew. %) | (Gew. %) | (Gew. %) |
| Hydroxyethylenmethacrylat | 49,7 | 39,7 | 35,7 | 45,7 |
| Phosphatdi methacrylat [Bis(glyceryl dimethacrylate) phosphate] | 49,7 | 59,7 | 54,6 | 44,7 |
| Campherchinon | 0,4 | 0,4 | 0,5 | 0,4 |
| Triethylamin | 0,2 | | 0,1 | 0,2 |
| N,N-Dimethyl-p-toluidin | | 0,2 | 0,1 | |

### B) Nagelspange (weich)

| | **B1** | **B2** | **B3** | **B4** | **B5** |
|---|---|---|---|---|---|
| **Komponente** | (Gew. %) | (Gew. %) | (Gew. %) | (Gew. %) | (Gew. %) |
| Bisphenol-(A)-(di)methacrylat | 16,0 | 15,0 | 14,0 | 30,0 | 22,0 |
| Urethan-dimethacrylat | 16,0 | 30,0 | 30,0 | 15,0 | 22,0 |
| Silica filler (Aerosil 9200) | 20,0 | 0,0 | 17,0 | 16,5 | 18,5 |
| Silica filler (Aerosil 7200) | 5,0 | 10,3 | 15,0 | 12,5 | 4,5 |
| Barium Glas (median particle size: 13µm) | 18,0 | 13,0 | 13,0 | 12,0 | 14,5,0 |
| Barium Glas (median particle size: 5µm) | 3,9 | 14,0 | 5,2 | 5,0 | 5,5 |
| Polymer Partikel (median particle size: 10µm) | 20,0 | 12,0 | 4,0 | 4,5 | 8,0 |
| Polymer Partikel (median particle size: 6µm) | 0,0 | 5,0 | 1,0 | 3,5 | 4,0 |
| Campherchinon | 0,6 | 0,4 | 0,5 | 0,6 | 0,0 |
| Triethylamin | 0,5 | 0,0 | 0,1 | 0,4 | 0,0 |
| N,N-Dimethyl-p-toluidin | 0,0 | 0,3 | 0,2 | 0,0 | 0,0 |
| 2-Ethyl-Anthrachinon | 0,0 | 0,0 | 0,0 | 0,0 | 0,6 |
| N-Phenyl Glycin | 0,0 | 0,0 | 0,0 | 0,0 | 0,4 |

### C) Nagelspange (hart)

| | **C1** | **C2** | **C3** | **C4** | **C5** |
|---|---|---|---|---|---|
| **Komponente** | (Gew. %) | (Gew. %) | (Gew. %) | (Gew. %) | (Gew. %) |
| Bisphenol-(A)-(di)methacrylat | 20,0 | 25,0 | 18,0 | 19,0 | 15,0 |
| Urethan-dimethacrylat | 20,0 | 13,0 | 20,0 | 20,0 | 23,0 |
| Silica filler (Aerosil 9200) | 20,0 | 0,0 | 18,0 | 16,5 | 19,5 |
| Silica filler (Aerosil 7200) | 5,0 | 13,3 | 16,0 | 15,5 | 5,5 |
| Barium Glas (median particle size: 13µm) | 16,0 | 14,0 | 12,5 | 12,0 | 12,5 |
| Barium Glas (median particle size: 5µm) | 3,9 | 16,1 | 7,0 | 6,5 | 8,5 |
| Polymer Partikel (median particle size: 10µm) | 14,0 | 12,0 | 6,2 | 6,0 | 11,0 |
| Polymer Partikel (median particle size: 6µm) | 0,0 | 5,0 | 1,0 | 3,5 | 4,0 |
| Campherchinon | 0,6 | 0,8 | 0,7 | 0,6 | 0,0 |
| Triethylamin | 0,5 | 0,0 | 0,2 | 0,4 | 0,0 |
| N,N-Dimethyl-p-toluidin | 0,0 | 0,8 | 0,4 | 0,0 | 0,0 |
| 2-Ethyl-Anthrachinon | 0,0 | 0,0 | 0,0 | 0,0 | 0,6 |
| N-Phenyl Glycin | 0,0 | 0,0 | 0,0 | 0,0 | 0,4 |

### Figuren

**Figur 1** zeigt die Auftragung einer Zusammensetzung zur Herstellung einer lichthärtenden Nagelspange auf dem Nagel.
**Figur 2** zeigt die lichtgehärtete Zusammensetzung auf dem Nagel nahe der Nageltasche (nach der Belichtung).
**Figur 3** zeigt die Auftragung einer lichthärtenden Zusammensetzung auf dem Nagel nahe der Nageltasche.
**Figur 4** zeigt die Anbringung einer Federspange mittels des erfindungsgemäßen Kits durch Fixierung des ersten Endes der Federspange (oben) und anschließende Fixierung des anderen Endes.

## Patentansprüche

1. Kit zur Korrektur eines Hand- oder Fußnagels, enthaltend
a) Grundierungsmittel (Primer), enthaltend
| | |
|---|---|
| 40-60% | Hydroxyethylenmethacrylat |
| 40-60% | Phosphatdimethacrylat, |
| 0,1-1,0 % | Starter, |
b) mindestens eine Zusammensetzung zur Herstellung einer lichthärtenden Nagelspange, enthaltend
| | |
|---|---|
| 15 -45 % | Bisphenol-(A)-(di)methacrylat, Urethan-dimethacrylat im Verhältnis 1:5 bis 5:1, |
| 85 - 55% | Füllstoffe und Pigmente |
| 0,1-1 % | Campherchinon, Aminostarter. |

2. Kit zur Korrektur eines Hand- oder Fußnagels gemäß Anspruch 1, zusätzlich enthaltend ein oder mehrere Federspangen.

3. Kit zur Korrektur eines Hand- oder Fußnagels gemäß Anspruch 1 oder 2, enthaltend
zwei Zusammensetzungen zur Herstellung von lichthärtenden Nagelspangen, nämlich eine erste Zusammensetzung enthaltend
| | |
|---|---|
| 17 -21 % | Bisphenol-(A)-(di)methacrylat, Urethan-dimethacrylat im Verhältnis 1:4 bis 4:1, |
| 79 - 83 % | Füllstoffe und Pigmente |
| 0,1-1 % | Campherchinon, Aminostarter, |
und eine zweite Zusammensetzung enthaltend
| | |
|---|---|
| 36 -40 % | Bisphenol-(A)-(di)methacrylat, Urethan-dimethacrylat im Verhältnis 1:4 bis 4:1, |
| 60 - 64 % | Füllstoffe und Pigmente |
| 0,1-1 % | Campherchinon, Aminostarter. |

4. Kit zur Korrektur eines Hand- oder Fußnagels gemäß Anspruch 1, 2 oder 3, **dadurch gekennzeichnet, dass** das Massenverhältnis von Bisphenol-(A)-(di)methacrylat zu Urethan-dimethacrylat in den Zusammensetzungen zur Herstellung von lichthärtenden Nagelspangen im Bereich von 1:2 bis 2:1, vorzugsweise 1:1 ist.

5. Kit zur Korrektur eines Hand- oder Fußnagels gemäß Anspruch 1 bis 4, **dadurch gekennzeichnet, dass** der Aminostarter 4-Dimethylaminobenzoesäureethylester ist.

6. Kit zur Korrektur eines Hand- oder Fußnagels gemäß Anspruch 1 bis 4, **dadurch gekennzeichnet, dass** der Primer 0,1 - 2 % mindestens eines Antimykotikums enthält.

7. Kit zur Korrektur eines Hand- oder Fußnagels gemäß Anspruch 1 bis 6, **dadurch gekennzeichnet, dass** mindestens ein Antimykotikum ausgewählt ist aus der Gruppe Econazole, Bifonazole, Chlodrimazole, Fenticonazole, Ketocanazole, Miconazole, Oxiconazole.

## Claims

1. A kit for the correction of a fingernail or a toenail, including
a) a primer, including
40 - 60 % of hydroxyethylene methacrylate
40 - 60 % of phosphate dimethacrylate,
0.1 - 1.0 % of a starter,
b) at least one composition for making a light-curing nail brace, including
15 - 45 % of bisphenol (A) (di)methacrylate, urethane dimethacrylate in a proportion from 1:5 to 5:1,
85 - 55 % of fillers and pigments
0.1 - 1 % of camphorquinone, amino starter.

2. The kit for the correction of a fingernail or toenail according to claim 1, additionally including one or more spring braces.

3. The kit for the correction of a fingernail or toenail according to claim 1 or 2, including
two compositions for making light-curing nail braces, namely a first composition including
17 - 21 % of bisphenol (A) (di)methacrylate, urethane dimethacrylate in a proportion from 1:4 to 4 : 1,
79 - 83 % of fillers and pigments
0.1 - 1 % of camphorquinone, amino starter,
and a second composition including
36 - 40 % of bisphenol (A) (di)methacrylate, urethane dimethacrylate in a proportion from 1:4 to 4:1,
60 - 64 % of fillers and pigments,
0.1 - 1 % of camphorquinone, amino starter.

4. The kit for the correction of a fingernail or toenail according to claim 1, 2 or 3,
**characterized by** that the mass proportion of bisphenol (A) (di)methacrylate to urethane dimethacrylate in the compositions for making light-curing nail braces is in the range from 1:2 to 2:1, preferably 1:1.

5. The kit for the correction of a fingernail or toenail according to claim 1 to 4,
**characterized by** that the amino starter is 4-dimethylaminobenzoic acid ester.

6. The kit for the correction of a fingernail or toenail according to claim 1 to 4,
**characterized by** that the primer includes 0.1 - 2 % of at least one antimycotic.

7. The kit for the correction of a fingernail or toenail according to claim 1 to 6,
**characterized by** that at least one antimycotic is selected from the group of econazole, bifonazole, chlodrimazole, fenticonazole, ketocanazole, miconazole, oxiconazole.

## Revendications

1. Kit de correction d'un ongle du doigt ou d'un ongle du pied, comportant
a) un apprêt, comportant
40 - 60 % de hydroxyéthyléne méthacrylate
40 - 60 % de phosphate diméthacrylate,
0,1 - 1,0 % d'un produit de départ,
b) au moins une composition de fabrication d'une agrafe d'ongle durcissante à la lumière, comportant
15 - 45 % de bisphénol (A) (di)méthacrylate, uréthane diméthacrylate dans un rapport compris entre 1:5 et 5:1,
85 - 55 % de charges et pigments
0,1 - 1 % de camphorquinone, produit de départ amino.

2. Kit de correction d'un ongle du doigt ou d'un ongle du pied selon la revendication 1, en outre comportant une ou plusieurs agrafes à ressort.

3. Kit de correction d'un ongle du doigt ou d'un ongle du pied selon la revendication 1 ou 2, comportant
deux compositions de fabrication d'agrafes d'ongle durcissantes à la lumières, c'est-à-dire une première composition comportant
17 - 21 % de bisphénol (A) (di)méthacrylate, uréthane diméthacrylate dans un rapport compris entre 1:4 et 4:1,
79 - 83 % de charges et pigments
0,1 - 1 % de camphorquinone, produit de départ amino,
et une deuxième composition comportant
36 - 40 % de bisphénol (A) (di)méthacrylate, uréthane diméthacrylate dans un rapport compris entre 1:4 et 4:1,
60 - 64 % de charges et pigments,
0,1 - 1 % de camphorquinone, produit de départ amino.

4. Kit de correction d'un ongle du doigt ou d'un ongle du pied selon la revendication 1, 2 ou 3,
**caractérisé en ce que** le rapport massique de bisphénol (A) (di)méthacrylate à uréthane diméthacrylate dans les compositions de fabrication d'agrafes d'ongle durcissantes à la lumières est compris entre 1:2 et 2:1, de préférance 1:1.

5. Kit de correction d'un ongle du doigt ou d'un ongle du pied selon la revendication 1 à 4,
**caractérisé en ce que** le produit de départ amino est de l'acide 4-diméthylamino benzoïque ester.

6. Kit de correction d'un ongle du doigt ou d'un ongle du pied selon la revendication 1 à 4,
**caractérisé en ce que** l'apprêt comporte 0,1 - 2 % d'au moins un antimycosique.

7. Kit de correction d'un ongle du doigt ou d'un ongle du pied selon la revendication 1 à 6,
**caractérisé en ce qu'**au moins un antimycosique est choisi à partir du groupe d'éconazole, bifonazole, chlodrimazole, fenticonazole, kétocanazole, miconazole, oxiconazole.
